**Europäisches Patentamt**

**European Patent Office**

**Office européen des brevets**

(19)

(11) Veröffentlichungsnummer: **0 244 562
B1**

(12) # EUROPÄISCHE PATENTSCHRIFT

(45) Veröffentlichungstag der Patentschrift:
**15.11.89**

(51) Int. Cl.⁴: **A61B 6/00, A61B 6/10**

(21) Anmeldenummer: **87101372.8**

(22) Anmeldetag: **02.02.87**

(54) **Röntgendiagnostikgerät.**

(30) Priorität: **07.05.86 DE 3615481**

(43) Veröffentlichungstag der Anmeldung:
**11.11.87 Patentblatt 87/46**

(45) Bekanntmachung des Hinweises auf die Patenterteilung:
**15.11.89 Patentblatt 89/46**

(84) Benannte Vertragsstaaten:
**DE FR**

(56) Entgegenhaltungen:
**DE-A- 2 341 975
FR-A- 2 255 038
US-A- 3 025 401
US-A- 4 293 927**

(73) Patentinhaber: **Siemens Aktiengesellschaft,
Wittelsbacherplatz 2, D-8000 München 2(DE)**

(72) Erfinder: **Kaul, Karlheinz, Dipl.-Ing. (FH), Ruhsteinweg 4,
D-8525 Uttenreuth(DE)**
Erfinder: **Seuss, Eckard, Dipl.-Ing. (FH),
Johann-Sebastian-Bach-Strasse 5,
D-8523 Baiersdorf(DE)**

## Beschreibung

Die Erfindung betrifft ein Röntgendiagnostikgerät mit Bedienelementen zur Steuerung bestimmter Komponenten und einem Aktivierungsschalter durch den die Bedienelemente gemeinsam aktivierbar oder wirkungslos schaltbar sind. Ein Röntgendiagnostikgerät dieser Gattung ist aus US-A-3 025 401 bekannt.

Bei einem Röntgendiagnostikgerät kann als Röntgensystem ein C-Bogen mit einem Röntgenstrahler und einem Strahlenempfänger an seinen Enden vorgesehen sein. Ein Gerät dieser Art kann für die Angiokardiographie verwendet werden. Der zu untersuchende Bereich des Patienten wird dabei durch Einstellung des C-Bogens längs seiner Umfangsrichtung, durch Verschwenken des C-Bogens und durch Verfahren der Säule gewählt. Diese Bewegungen können an einem Bedienkästchen ferngesteuert werden, an dem Bedienelemente dafür angeordnet sind, die insbesondere von Kipphebeln gebildet sein können.

Der Erfindung liegt die Aufgabe zugrunde, ein Röntgendiagnostikgerät der eingangs genannten Art so auszubilden, daß beim versehentlichen Betätigen eines Bedienelementes, insbesondere eines Kipphebels, kein Steuervorgang ausgelöst wird, daß also insbesondere keine Geräteverstellung erfolgt, wenn eines der entsprechenden Bedienelemente versehentlich, z.B. beim Vorbeistreifen mit einem Kleidungsstück oder mit dem Arm betätigt wird.

Diese Aufgabe ist erfindungsgemäß dadurch gelöst, daß ein Zeitglied vorhanden ist, das die Bedienelemente während einer vorbestimmten Zeit nach dem Betätigen des Aktivierungsschalters aktiviert. Bei dem erfindungsgemäßen Röntgendiagnostikgerät muß der Benutzer vor dem Auslösen eines Steuervorganges den Aktivierungsschalter bewußt betätigen. Der Aktivierungsschalter aktiviert dabei die Bedienelemente während einer vorbestimmten Zeit, so daß sie nur in dieser Zeit auch Steuervorgänge auslösen. Unbeabsichtigte Steuervorgänge sind praktisch verhindert.

Eine besonders zweckmäßige Ausbildung ergibt sich, wenn der Aktivierungsschalter zusammen mit den Bedienelementen an einem Bedienkästchen angebracht ist, welches am Patientenlagerungstisch angeordnet ist.

Die Erfindung ist nachfolgend anhand eines in der Zeichnung dargestellten Ausführungsbeispieles näher erläutert. Es zeigen:

Fig. 1 ein Röntgendiagnostikgerät nach der Erfindung, und
Fig. 2 ein Schaltbild der für die Erfindung wesentlichen Teile des Röntgendiagnostikgerätes gemäß Figur 1.

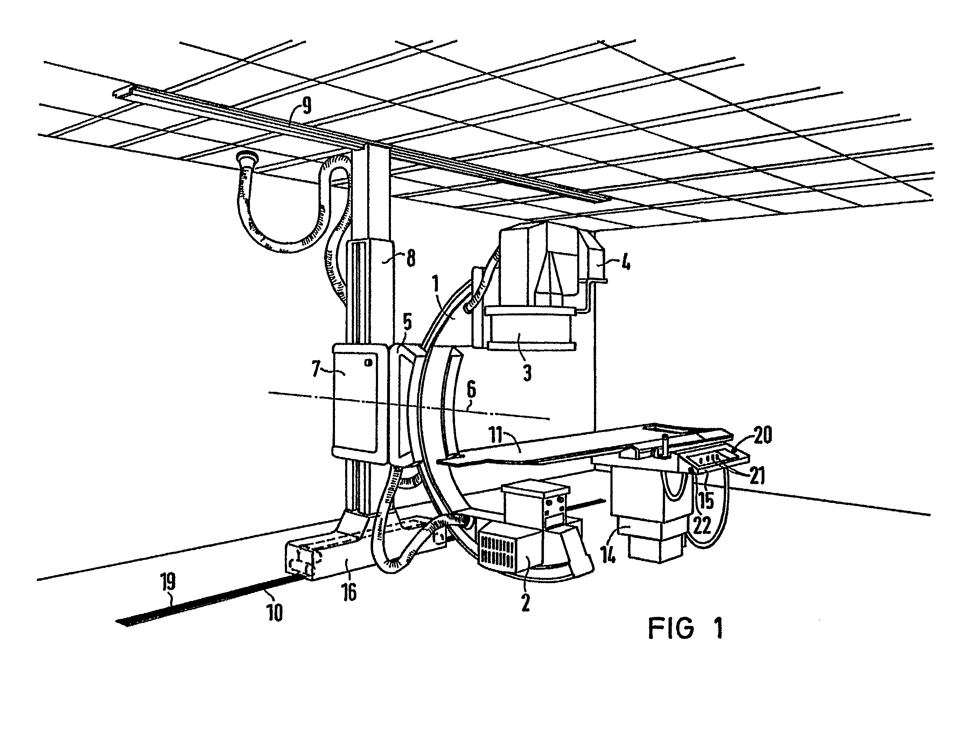

In der Figur 1 ist ein C-Bogen 1 dargestellt, der an seinen Enden einen Röntgenstrahler 2 und einen Röntgenbildverstärker 3 mit nachgeschalteter Einzelbildkamera 4 sowie nicht sichtbarer Fernsehkamera trägt. Der C-Bogen 1 ist längs seines Umfanges an einem Halter 5 verstellbar gelagert, welcher um eine horizontale Achse 6 schwenkbar mit einem Wagen 7 verbunden ist. Der Wagen 7 ist an einer vertikalen Säule 8 längsverschiebbar gelagert, welche in einer Deckenschiene 9 und einer Bodenschiene 10, welche parallel zueinander und zur Längsrichtung eines Patientenlagerungstisches 11 verlaufen, verschiebbar ist. Der C-Bogen 1 umgreift den Patientenlagerungstisch 11 quer. Dieser ist auf einem Sockel 14 höhenverstellbar gelagert, an dem ein Bedienpult 15 befestigt ist. Er ist gegenüber dem Sockel 14 in Längs- und Querrichtung verstellbar (schwimmende Lagerung), wobei jedoch der Stellweg in Längsrichtung relativ klein ist (Größenordnung 40 cm).

Die Wahl des untersuchten Bereiches erfolgt im wesentlichen durch Einstellung des Röntgenstrahlers 2 und des Röntgenbildverstärkers 3, also nicht durch Verstellung des Patientenlagerungstisches 11. Die Einstellung kann dabei in folgender Weise erfolgen:

Zunächst wird die Säule 8 motorisch ganz nach links verfahren. In der so erreichten Stellung wird der C-Bogen 1 in die jeweilige Untersuchungslage gebracht (Bildverstärker 3 oberhalb oder unterhalb des Patientenlagerungstisches 11 oder seitlich von diesem). Anschließend wird die Säule 8 so weit zurückgefahren, bis der Röntgenstrahler 2 und der Röntgenbildverstärker 3 unter bzw. über dem zu durchstrahlenden Bereich des Patienten oder seitlich an diesem liegen. Die jeweilige Höhenlage des Isozentrums wird dabei über den Wagen 7 eingestellt. Schließlich kann die Strahlenrichtung auch noch durch Schwenken des C-Bogens 1 um die Achse 6 variiert werden.

Die Säule 8 ist an ihrer Unterseite mit einem Wagen 16 verbunden, der mit Rädern in der Schiene 10 geführt ist. Die Schiene 10 ist von einem oben offenen, geschlitzten Hohlprofil gebildet, in dem diese Räder laufen. Die offene Oberseite der Schiene 10 ist von einem flexiblen Band 19 abgedeckt, das durch den Wagen 16 verläuft und demgemäß das Hineinfallen von Gegenständen in das Innere der Schiene 10 verhindert.

Die Steuerung der einzelnen Komponenten des Röntgendiagnostikgerätes gemäß Figur 1 erfolgt durch Bedienelemente an einem Bedienkästchen 20, welches am Patientenlagerungstisch 11 angebracht ist. Das Bedienkästchen 20 trägt dabei insbesondere Kipphebel 21, welche die verschiedenen elektromotorischen Bewegungen der einzelnen Komponenten des Röntgendiagnostikgerätes steuern. Die Kipphebel 21 können versehentlich durch eine am Bedienkästchen 20 vorbeigehende Person betätigt werden. Damit dabei keine Steuervorgänge ausgelöst werden, ist an der linken Stirnseite des Bedienkästchens 20 eine Aktivierungstaste 22 vorhanden. Wird die Aktivierungstaste 22 betätigt, so werden die Kipphebel 21 während einer vorbestimmten Zeit von beispielsweise 20 Sekunden aktiviert. Dies bedeutet, daß nur innerhalb dieser Zeit nach Betätigung der Aktivierungstaste 22 Steuervorgänge mit Hilfe der Kipphebel 21 ausgelöst werden können. Ist die Zeit von 20 Sekunden nach dem Betätigen der Aktivierungstaste 22 verstrichen, so ist das Betätigen der Kipphebel 21 wirkungslos.

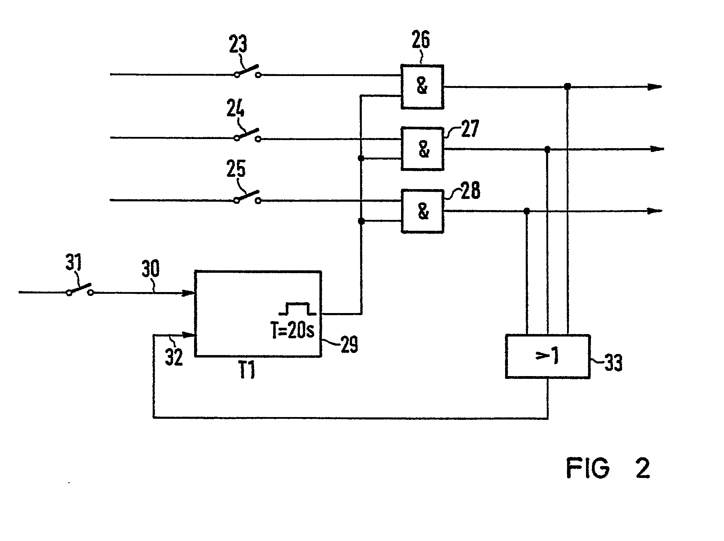

In der Figur 2 sind drei Kontakte 23, 24, 25 dargestellt, die durch drei der Kipphebel 21 betätigt werden. Die Kontakte 23 bis 25 sind an drei UND-Gattern 26, 27, 28 angeschlossen, deren zweite Eingänge zu einem Zeitgeber 29 führen. Der Zeitgeber 29 besitzt einen Eingang 30, der zu einem Schalter 31 führt, welcher durch die Aktivierungstaste 22 betätigbar ist. Der zweite Eingang 32 ist über ein ODER-Gatter 33 an den Ausgängen der UND-Gatter 26, 27, 28 angeschlossen, die zur Steuerelektronik führen.

Wird die Aktivierungstaste 22 betätigt und damit der Schalter 31 geschlossen, so wird das Zeitglied 29 getriggert und die Schalter 23, 24, 25 während einer Zeit von 20 Sekunden zur Gerätesteuerung freigegeben. Wird innerhalb dieser Zeit einer der Schalter 23, 24, 25 betätigt, so wird am entsprechenden UND-Gatter 26, 27, 28 ein Ausgangsimpuls erzeugt, der über das ODER-Gatter 33 das Zeitglied 29 derart ansteuert, daß von dem Betätigungszeitpunkt ab erneut eine Zeitspanne von 20 Sekunden läuft, innerhalb der einer der Schalter 23, 24, 25 zur Gerätesteuerung freigegeben ist. Wird also innerhalb einer Zeit von 20 Sekunden nach Betätigen des Schalters 31 25 und gegebenenfalls nachfolgend eines der Schalter 23, 24, weder die Aktivierungstaste 22 (Schließen des Schalters 31) noch einer der Schalter 23, 24, 25 betätigt, so fällt das System in den Ruhezustand zurück und muß erneut aktiviert werden.

Die geschilderten Funktionen können auch per Software realisiert werden.

## Patentansprüche

1. Röntgendiagnostikgerät mit Bedienelementen (21, 23, 24, 25) zur Steuerung bestimmter Komponenten (1 bis 8) und einem Aktivierungsschalter (22, 31) durch den die Bedienelemente (21, 23, 24, 25) gemeinsam aktivierbar oder wirkungslos schaltbar sind, **gekennzeichnet durch** ein Zeitglied (29), das die Bedienelemente (21, 23, 24, 25) während einer vorbestimmten Zeit nach dem Betätigen des Aktivierungsschalters (22, 31) aktiviert.

2. Röntgendiagnostikgerät nach Anspruch 1, **dadurch gekennzeichnet**, daß der Aktivierungsschalter (22, 31) zusammen mit den Bedienelementen (21, 23, 24, 25) an einem Bedienkästchen (20) angebracht ist, das an einem Patientenlagerungstisch (11) angeordnet ist.

3. Röntgendiagnostikgerät nach Anspruch 1, **dadurch gekennzeichnet**, daß dem Zeitglied (29) auch Steuersignale von den Bedienelementen (21, 23, 24, 25) zur Freigabe der Bedienelemente (21, 23, 24, 25) während einer vorbestimmten Zeit nach dem letzten wirksamen Betätigen eines der Bedienelemente (21, 23, 24, 25) zugeführt werden.

## Claims

1. X-ray diagnostic equipment having operating elements (21, 23, 24, 25) for the control of certain components (1 to 8) and having an activation switch (22, 31), by means of which the operating elements (21, 23, 24, 25) can be jointly switched so that they become activatable or ineffective, characterised by a timing element (29) which activates the operating elements (21, 23, 24, 25) during a predetermined time after actuation of the activation switch (22, 31).

2. X-ray diagnostic equipment according to claim 1, characterised in that the activation switch (22, 31) is provided, together with the operating elements (21, 23, 24, 25), on an operating box (20) which is arranged on a patient positioning table (11).

3. X-ray diagnostic equipment according to claim 1, characterised in that control signals are also supplied to the timing element (29) from the operating elements (21, 23, 24, 25) for the release of the operating elements (21, 23, 24, 25) during a predetermined time after the last effective actuation of one of the operating elements (21, 23, 24, 25).

## Revendications

1. Appareil de radiodiagnostic comportant des éléments de commande (21, 23, 24, 25) servant à commander des composants déterminés (1 à 8), et un interrupteur d'activation (22, 31), à l'aide duquel les éléments de commande (21, 23, 24, 25) peuvent être activés ou rendus inactifs, en commun, caractérisé par une minuterie (29), qui active les éléments de commande (21, 23, 24, 25) pendant un intervalle de temps prédéterminé, après l'actionnement de l'interrupteur d'activation (22, 31).

2. Appareil de radiodiagnostic suivant la revendication 1, caractérisé par le fait que l'interrupteur d'activation (22, 31) est disposé, ainsi que les éléments de commande (21, 23, 24, 25), sur un petit boîtier de commande (20), qui est disposé sur un plateau (11) formant couchette pour patient.

3. Appareil de radiodiagnostic suivant la revendication 1, caractérisé par le fait que des signaux de commande sont également envoyés à la minuterie (29) par les éléments de commande (21, 23, 24, 25) pour la libération de ces derniers pendant un intervalle de temps prédéterminé, après la dernière activation effective de l'un des éléments de commande (21, 23, 24, 25).

FIG 1

EP 0 244 562 B1

FIG 2